Europäisches Patentamt

European Patent Office

Office européen des brevets

(11) Veröffentlichungsnummer: **0 074 620**

**A2**

(19)

(12) # EUROPÄISCHE PATENTANMELDUNG

(21) Anmeldenummer: **82108306.0**

(22) Anmeldetag: **09.09.82**

(51) Int. Cl.³: **A 61 K 47/00**
**A 61 K 9/08, C 09 K 15/00**

(30) Priorität: **12.09.81 DE 3136282**

(43) Veröffentlichungstag der Anmeldung:
**23.03.83 Patentblatt 83/12**

(84) Benannte Vertragsstaaten:
**AT CH DE FR GB IT LI**

(71) Anmelder: **HOECHST AKTIENGESELLSCHAFT**
**Postfach 80 03 20**
**D-6230 Frankfurt/Main 80(DE)**

(72) Erfinder: **Thoma, Karl, Dr. Prof.**
**Hochkalterstrasse 10**
**D-8000 München 90(DE)**

(54) **Verfahren zur Stabilisierung photoinstabiler Arzneistoffe sowie stabilisierte Arzneizubereitungen.**

(57) Stabilisierung von photoinstabilen Arzneistoffen durch Zusatz von pharmakologisch verträglichen, spektral absorbierenden Substanzen, die ein ähnliches Absorptionsverhalten aufweisen wie die zu schützenden Arzneistoffe.

EP 0 074 620 A2

HOECHST AKTIENGESELLSCHAFT HOE 81/F 244    Dr. D/Fr

Verfahren zur Stabilisierung photoinstabiler Arzneistoffe
sowie stabilisierte Arzneizubereitungen

Die vorliegende Erfindung betrifft die Stabilisierung
photoinstabiler Arzneistoffe gegen den zersetzenden
Einfluß von UV-Licht und Tageslicht.
Zahlreiche Arzneistoffe sind instabil gegen den Einfluß
von Tageslicht (photoinstabil).

Als Beispiele photoinstabiler Arzneistoffe seien hier
nur einige genannt: Chlorpromazin, Triflupromazin,
Adrenalin, Menadion, Nifedipin, Nitrofurazon, Xanthocillin,
Nitroprussid-Natrium, Riboflavin, Reserpin, Chloramphenicol,
Dihydroergotamin, Furosemid, Piretanid und Chlorprothixin.

Komplizierte chemische Strukturen erhöhen häufig die
Photoinstabilität von arzneilich verwendeten Substanzen.

Insbesondere die Lösungen solcher Stoffe weisen eine in
der pharmzeutischen Praxis nicht tolerierbare Anfälligkeit gegenüber Licht auf.

Die wirksamste Methode photolabile Arzneistoffe und Zubereitungen gegen Lichteinfluß zu schützen, ist die Verwendung einer lichtundurchlässigen Verpackung. In der
Praxis kann dadurch jedoch nur für den Zeitraum der
Lagerung eines Arzneimittels völliger Lichtabschluß gewährleistet werden.

Während des Herstellungsprozesses und der Applikation
lassen sich dagegen Lichtexpositionen nicht ausschließen.
Für diesen Zeitraum können daher weitergehende, von der
Verpackung unabhängige Stabilisierungsmaßnahmen notwendig
werden.

Im Mittelpunkt solcher Anstrengungen stehen dabei die arzneilichen Lösungen, da diese generell gegenüber lichtinduzierten Umsetzungen besonders gefährdet sind und darüber hinaus die zur Überprüfung der Dosierung und Reinheitskontrolle notwendige Sichtkontrolle eine Verwendung undurchsichtiger Primärpackmittel häufig ausschließt.

Kompromißlösungen mit durchaus überzeugenden Stabilisierungseigenschaften stellen gefärbte Gläser dar, durch welche jedoch lediglich die energiereichen Wellenlängen kleiner als 500 nm absorbiert werden. Zur Beurteilung ihrer Verwendbarkeit haben Anforderungen an die Lichtdurchlässigkeit solcher Behältnisse Eingang in zahlreiche Arzneibücher gefunden. Ihre Verwendung für Injektionslösungen ist allerdings durch die Notwendigkeit einer nachträglichen Sichtkontrolle auf Schwebeteilchen eingeschränkt. Die Pharmacopée Française schreibt deshalb für Injektionsgläser den Einsatz von farblosem Glas vor. In solchen Fällen ist die Wahl alternativer Stabilisierungsmaßnahmen notwendig.

Es wurden schon mehrere solche alternative Stabilisierungsmöglichkeiten beschrieben. Welche dieser Möglichkeiten jeweils zweckmäßigerweise angewandt wird, ist im Einzelfall abhängig von der Art des photolytischen Vorgangs. So können lichtinduzierte Redoxreaktionen durch die Zugabe von Redoxstabilisatoren zurückgedrängt werden. Beispiele hierfür sind die Stabilisierung von Nystatin-Lösungen mit Butylhydroxyanisol und von Sulfacetamid-Natriumlösungen durch Zugabe von Natriumthiosulfat.

Durch Bildung von "Charge-Transfer"-Komplexen zwischen photoinstabilen Substanzen und geeigneten Komplexbildnern können labile Elektronsysteme stabilisiert werden.

Zur Stabilisierung von Arzneimitteln, bei welchen die photolytische Zersetzung über die Bildung langlebiger Radikale verläuft, können sogenannte Radikalfänger (Quencher) eingesetzt werden. So wird die Lichtstabilität von Riboflavin durch Zusatz von Kaliumjodid wesentlich erhöht.

Es wurde auch schon versucht, die Photostabilität von Farbstoffen in Tabletten oder Drageeüberzügen durch Zusatz von Lichtschutzmitteln, die in dermatischen Zubereitungen verwendet werden, zu verbessern. Die Erfolge waren wenig befriedigend, da sich die Schutzwirkung nur auf den dermatologisch relevanten UV-Bereich beschränkt (vgl. z.B. Journal of Pharmaceutical Sciences 51, 321 (1962), 63 129 (1974), 63, 1927 (1974)).

Es wurde nun überraschenderweise gefunden, daß photoinstabile Arzneistoffe in Arzneiformen durch Zusatzsubstanzen, welche ein ähnliches Absorptionsverhalten wie die zu schützenden Arzneistoffe aufweisen, unter bestimmten Voraussetzungen stabilisiert werden können.

Die Erfindung betrifft daher ein Verfahren zum Stabilisieren von photoinstabilen Arzneistoffen in Arzneiformen, welches dadurch gekennzeichnet ist, daß man geeignete pharmakologisch verträgliche spektral absorbierende Substanzen, welche ein ähnliches Absorptionsverhalten wie die zu schützenden Arzneistoffe aufweisen und vor dem jeweils speziellen, die Photoinstabilität auslösenden Spektralbereich des Lichtes schützen, zusetzt.

Als Arzneiformen kommen z.B. Lösungen für verschiedene Anwendungsbereiche, Kapseln, Hydrogele, Pulvermischungen, Granulate und Komprimate in Betracht.

Die Erfindung betrifft daher auch pharmazeutische Präparate enthaltend nach dem erfindungsgemäßen Verfahren stabilisierte Arzneistoffe.

Bei dem erfindungsgemäßen Verfahren wird der photoinstabile Arzneistoff dadurch stabilisiert, daß ein gelöster Stoff zugegeben wird, der ein ähnliches Absorptionsverhalten gegenüber Licht wie die zu schützende Substanz aufweist. Dieser gelöste Stoff wird beispielsweise dem Wirkstoffpulver zugesetzt und anschließend wird das Gemisch nach herkömmlichen Methoden zu Tabletten verpreßt, oder in Kapseln gefüllt. Es ist auch möglich z.B. der fertigen Lösung eines Arzneistoffes die Lösung der Schutzsubstanz zuzusetzen. Der Photostabilisator wird zweckmäßig zu einem frühen Zeitpunkt dem instabilen Arzneistoff zugesetzt.

Als Photostabilisatoren kommen solche Substanzen in Betracht, die ein ähnliches Absorptionsverhalten aufweisen wie der photolabile Arzneistoff. Die Stabilisatoren können einzeln oder im Gemisch vorliegen. Das Verhältnis zwischen Stabilisator und Arzneistoff variiert und hängt u.a. ab von der gewünschten Verbesserung und der Verträglichkeit des Stabilisators.

Im UV-Bereich von 290 bis 400 nm werden instabile Arzneistoffe mit Verbindungen wie Vanillin, Procain, 8-OH-Chinolin, Benzylalkohol, 4-OH-Benzoesäure, p-Amino-benzoesäure, Gallussäureestern oder deren Gemischen stabilisiert, welche im Bereich von 200 bis 300/350 nm ein deutliches Absorptionsvermögen besitzen. Mit Hilfe solcher Stoffe können Verbindungen wie Chlorpromazin und andere Phenothiazine, Menadion, Adrenalin, Reserpin, Chloramphenicol stabilisiert werden.

Tabelle 1 Stabilitätsverbesserung von Chlorpromazinlösungen durch Vanillin (Bestrahlungsdauer
24 Stunden, UVIS$^R$-Lampe, Fa. Desaga)

| Chlorpromazin (%) | Vanillin (%) | Stabilitätsverbesserung (%) |
|---|---|---|
| 0,2 | 0,02 | 74,6 |
| 0,2 | 0,1 | 83,6 |
| 0,2 | 0,2 | 92,7 |
| 0,2 | 0,5 | 109,5 |
| 0,4 | 0,02 | 33,2 |
| 0,4 | 0,1 | 39,6 |
| 0,4 | 0,2 | 49,2 |
| 0,4 | 0,5 | 64,5 |
| 1,0 | 0,02 | 11,6 |
| 1,0 | 0,1 | 17,2 |
| 1,0 | 0,2 | 26,2 |
| 1,0 | 0,5 | 28,9 |

Speziell niedere Chlorpromazinkonzentrationen können sehr
wirkungsvoll mit 0,5 % Vanillin stabilisiert werden.

Tabelle 2 Stabilitätsverbesserung von Chlorpromazinlösungen durch 8-OH-Chinolin (Bestrahlungsdauer 24 Stunden, UVIS$^R$-Lampe, Fa. Desaga)

| Chlorpromazin (%) | 8-Hydroxychinolin (%) | Stabilitätsverbesserung (%) |
|---|---|---|
| 0,2 | 0,01 | 70,9 |
| 0,2 | 0,05 | 80,5 |
| 0,2 | 0,10 | 95,7 |
| 0,4 | 0,01 | 34,4 |
| 0,4 | 0,05 | 39,9 |
| 0,4 | 0,10 | 51,0 |
| 1,0 | 0,01 | 21,3 |
| 1,0 | 0,05 | 22,0 |
| 1,0 | 0,10 | 28,2 |

Tabelle 3 Photostabilisierung von Menadionsolubilisaten mit Hilfe von Vanillin (5 mg/100 ml; Grundlösung Phosphatpuffer pH 5,9; 5% Polyäthylenglycol(20) glycerinoleat)

| Stabilisator | Gehalt nach 75 Min. |
|---|---|
| kein Zusatz | 8,9 % |
| 0,2 % Vanillin | 91,6 % |

Tabelle 4 Stabilitätsverbesserung von Piretanid-Lösung (75 mg Piretanid + 435 mg NaCl + $H_2O$/NaOH ad 50 ml/pH 9) mit Hilfe von Vanillin; einem Gemisch aus Vanillin und 4-OH-Benzoesäure; Benzylalkohol 4-OH-Benzoesäure (Bestrahlungsdauer 4 Stunden im Lichtprüfschrank nach Thoma /Strittmatter)

| Stabilisator | Gehalt an Piretanid in % nach | | | | |
|---|---|---|---|---|---|
| | 0 | 60 | 120 | 180 | 240 Min. |
| kein Zusatz | 100 | 58 | 27,5 | 24 | - |
| Vanillin 100 mg% + 4-OH-Benzoesäure 50 mg% | 100 | 99 | 98 | 96 | 96 |
| Vanillin 75 mg% | 100 | 97.5 | 96 | 95 | 95 |
| Benzylalkohol 1 % | 100 | 83 | 76 | 72 | 65 |
| 4-OH-Benzoesäure 75 mg% | 100 | 90 | 82 | 67 | 56 |

Die Lichtstabilisierung von Piretanid kann, wie aus Tabelle 4 hervorgeht, besonders erfolgreich mit Vanillin sowie mit einem Gemisch aus Vanillin und 4-OH-Benzoesäure durchgeführt werden.

Ein Zusatz des in Injectabilia häufiger verwendeten Konservierungsmittels Benzylalkohol sowie ein solcher von 4-OH-Benzoesäure besitzt einen weniger ausgeprägten, jedoch signifikanten Stabilisierungseffekt.

Arzneistoffe, die im sichtbaren Bereich des Lichtes von 400 bis 700 nm instabil sind, können durch gezielte Anwendung von geeigneten Lebensmittelfarbstoffen oder anderen gefärbten Verbindungen stabilisiert werden.

Verbindungen, die mit Hilfe solcher Stabilisatoren geschützt werden können, sind z.B. Daunorubicin, Nifedipin, Riboflavin, Methylenblau sowie Xanthocillin, Nitrofurazon und Nitroprussid-Natrium.

Tabelle 5  Stabilitätsverbesserung von Nifedipin in wäßrig/äthanolischen Lösungen in Gegenwart von Tageslicht

| 16 mg% Nifedipin in Lösung und Zusatz von | Wirkstoffgehalt in % nach Stunden | | | | |
|---|---|---|---|---|---|
| | 1 | 2 | 3 | 4 | 5 |
| ohne | 10 | – | – | – | – |
| 16 mg% Chrysoin S | 95,4 | 80,3 | 75,3 | 65,8 | 60,4 |
| 16 mg% Echtgelb | 93 | 78,1 | 70 | 62,9 | 56 |
| 16 mg% Gelborange S | 68,5 | 44 | 28,6 | 18 | 10,6 |
| 16 mg% Tartrazin | 61,3 | 36,5 | 22,7 | 13,8 | – |
| 16 mg% Orange GGN | 58,7 | 30 | 18 | – | – |
| 16 mg% Chinolingelb | 50,5 | 24 | 11,8 | – | – |
| 16 mg% Cochenillerot A | 18 | – | – | – | – |
| 0,5 % 8-OH-Chinolinsulf. | 90,3 | 66 | 60,1 | 50 | 45,6 |

Die Stabilisierung photoinstabiler Wirkstoffe kann nach dem genannten Verfahren nicht nur in wäßrigen, alkoholischen oder anderen Lösungen durchgeführt werden, sondern läßt sich auch in Arzneiformen wie Tabletten, Kapseln, Salben, Suspensionen und anderen Arzneizubereitungen durchführen.

Tabelle 6  Stabilisierung von Nifedipin in Gelatine-Kapseln
mit Hilfe des gelben Lebensmittelfarbstoffs E 105
(Lichtstabilitäts-Prüfgerät Spectrotest,
Original HANAU)

| 8 mg Nifedipin in in Kapseln und Zusatz von | Wirkstoffgehalt in % nach Stunden | | | | | |
|---|---|---|---|---|---|---|
| | 1 | 2 | 3 | 4 | 5 | 6 |
| ohne | 19,1 | – | – | – | – | – |
| 0,5 % E 105 in Kaps. Hülle | 97,3 | 94,6 | 91,9 | 89,2 | 81,1 | – |
| 1,2 % E 105 in Kaps. Füllg. | 97 | 94,5 | 91 | 89 | 81,3 | – |
| E105 in Hülle u.Füllg. | 100 | 100 | 100 | 100 | 100 | 100 |

Tabelle 7  Stabilisierung von Nifedipin in Tabletten
mit Hilfe eines gelben Lebensmittelfarbstoffes
in Gegenwart von Tageslicht

| 4 mg Nifedipin in Tabletten und Zusatz von | Wirkstoffgehalt nach Tagen in % | | | | |
|---|---|---|---|---|---|
| | 5 | 10 | 15 | 20 | 25 |
| ohne | 80,3 | 72,6 | 71,8 | 71,5 | 71,2 |
| E 105 4 mg/Tabl. | 95,4 | 95,3 | 95,1 | 95 | 95 |

Tabelle 8  Stabilisierung von Nifedipin in Hydrogel-
Salben mit Hilfe des gelben Lebensmittelfarbstoffes E 100 (Lichtstabilitäts-Prüfgerät
Spectrotest, Original HANAU)

| 0,3 % Nifedipin in Hydrogelsalben | Wirkstoffgehalt nach Minuten in % | | | |
|---|---|---|---|---|
| | 60 | 120 | 180 | 300 |
| ohne | 50,1 | 25,3 | – | – |
| 0,3 % E 100 | 90,6 | 77,1 | 67,1 | 52,8 |

Tabelle 9  Stabilisierung von Riboflavin in Lösungen mit Hilfe des Lebensmittelfarbstoffes Tartrazin (Lichtstabilitäts-Prüfgerät Suntest, Original HANAU)

| t (min) | Restgehalt in % | |
|---|---|---|
| | ohne Zusatz | mit Stabilisator |
| 2 | 74 | 90 |
| 3 | 61 | 85,7 |
| 4 | 50,3 | 81,8 |
| 5 | 39 | 76,6 |

Tabelle 10  Stabilisierung von Daunorubicin in Lösungen mit Hilfe von roten und gelben Lebensmittelfarbstoffen (Lichtstabilitäts-Prüfgerät Spectrotest, Original HANAU)

| | Restgehalt in % nach Stunden | | | | | |
|---|---|---|---|---|---|---|
| | 1 | 2 | 3 | 4 | 5 | 6 |
| ohne Zusatz | 89 | 79,1 | 70 | 58,3 | 48 | 39,5 |
| + E 125 | 99 | 98,4 | 97,5 | 97 | 96,5 | 96 |
| + E 123 | 98,3 | 97 | 96 | 95,2 | 94,6 | 93,5 |
| + E 126 | 97 | 95 | 92,8 | 90 | 88 | 86 |
| + E 102 [*] | 95,7 | 88 | 80,3 | 74,1 | 70 | 65 |

[*] bewußt gewähltes Negativbeispiel mit relativ ungünstigem Absorptionsverhalten im Beispiel Daunorubicin

Tabelle .11 Stabilisierung von Methylenblau in Lösungen mit Hilfe von blauen und gelben Lebensmittel farbstoffen (Lichtstabilitäts-Prüfgerät Spectrotest, Original HANAU)

|  | Restgehalt in % nach Stunden | | | | |
|---|---|---|---|---|---|
|  | 1 | 2 | 3 | 4 | 5 |
| ohne Zusatz | 95,3 | 90 | 84,2 | 79,5 | 72,5 |
| + E 132 | 99 | 97,5 | 96,5 | 95,5 | 94 |
| + E 103 [*)] | 96,5 | 93 | 89,5 | 85,5 | 82 |

*) bewußt gewähltes Negativbeispiel mit relativ ungünstigem Absorptionsverhalten im Beispiel
Methylenblau

Außer den vorstehend erwähnten photolabilen Stoffen wurden die in Tabelle 12 zusammengestellten Arzneistoffe in Form von Lösungen nach dem erfindungsgemäßen Verfahren stabilisiert. Die Tabelle gibt Auskunft über den angewandten Stabilisator und den erreichten Effekt.

Die Untersuchungen zur Photostabilität und Stabilisierung wurden im wesentlichen wie folgt durchgeführt:

Zunächst wurden die Absorptionseigenschaften der Wirkstoffe und einiger Stabilisatoren mit bekanntermaßen ähnlichen Absorptionseigenschaften in vorzugweise methanolischer oder wäßriger Lösung untersucht. Anschließend wurde dann der Einfluß der verschiedenen Photostabilisatoren auf die Stabilität der Wirkstofflösung (Stammlösung) festgestellt, indem sowohl die Stammlösung als auch die Stammlösung mit Stabilisatorzusatz im Lichtprüfschrank (gefiltertes Quecksilberhochdrucklicht) belichtet wurden. Derjenige Stabilisator bzw. diejenige Stabilisatorlösung, mit der der günstigste Stabilisierungseffekt bewirkt wurde, wurde dann z.B. einer Injektionslösung zugesetzt, Injektionslösung mit und ohne Stabilisatorzusatz wurden dann ebenfalls im Lichtprüfschrank bestrahlt und die Konzentration an Wirkstoff in Abhängigkeit von der Bestrahlungsdauer bestimmt.

Tabelle 12 Stabilisierungserfolge bei photoinstabilen Arzneistoffen in Lösung durch Zusatz von Photostabilisatoren

| Arzneistoff (Konz.) | | Photostabilisator (Konz.) | Haltbarkeitsdauer [*] | |
|---|---|---|---|---|
| | | | unstabili. | stabilisiert |
| Dihydroergotamin-Methansulfonat (1 mg/ml) | Injektionslösung | Methylgallat (1 mg/ml) | 5 Std. | 70 Std. |
| Nitrofurazon (2 mg/ml) | Otalgicum | Kurkumin (0,5 mg/ml) | 7 Std. | 22 Std. |
| Thiothixen (2 mg/ml) | wäßrige Lösung | 8-Hydroxy-chinolin-sulfat/Vanillin (je 3 mg/ml) | 10 Min. | 40 Min. |
| Furosemid (10 mg/ml) | Injektionslösung | Vanillin (10 mg/ml) | 1,5 Std. | 7 Std. |
| Nitroprussid-Natrium (2,5 mg/ml) | | Trypanrot (5 mg/ml | — | — [**] |
| Haloperidol (5 mg/ml) | Injektionslösung | Vanillin/Benzylalkohol (je 5 mg/ml) | 25 Std. | 120 Std. |
| Fluphenazin (1 mg/ml) | Injektionslösung | Vanillin-Kurkumin (je 1 mg/ml) | 10 Min. | 60 Min. |
| Reserpin (0,25 mg/ml) | Injektionslösung | Propylgallat (0,25 mg/ml) | — | — [**] |
| Rescinnamin (1 mg/ml) | | Propylgallat (1 mg/ml) | 5 Min. | 40 Min. |

[*] Bezogen auf die Bestrahlung der Arzneistofflösungen in Quarzküvetten im Lichtprüfschrank und eine maximale Zersetzung von 10 %.

[**] Deutliche Stabilisierungsverbesserungen treten erst bei Zersetzungsraten größer als 10 % auf.

Aus Tabelle 12 ist ersichtlich, daß bei den Arzneistoffen Dihydroergotamin-Methansulfonat, Nitrofurazon und Furosemid eine erhebliche Stabilitätserhöhung mit den angegebenen Photostabilisatoren bewirkt wurde. Durch Verlängerung der Haltbarkeit um etwa eine Stunde können während der Herstellung und Applikation gravierende lichtinduzierte Wertminderungen vermieden werden.

Durch die erfindungsgemäße Stabilisierung ist es ferner möglich bei Arzneistoffen mit hoher Eigenstabilität eine lichtinduzierte Veränderung in der Praxis ganz auszuschließen.

Das erfindungsgemäße Stabilisierungsverfahren eignet sich auch für die Stabilisierung photoinstabiler Antidepressiva- und Neuroleptika-Lösungen. Als besonders instabil erwiesen sich Doxepin-HCl, Protriptylin-HCl, Flupentixol-di-HCl, Tiotixen-di-HCl, Chlorprothixen-HCl und Clopenthixol-di-HCl. In gleicher Weise, wie oben angegeben, wurden die Absorptionseigenschaften dieser 6 Verbindungen sowie der Stabilisatoren 8-Hydroxychinolin, Gallussäurepropylester und Vanillin bestimmt. Sodann wurde der Einfluß dieser Stabilisatoren auf die Stabilität der genannten Verbindungen untersucht in ähnlicher Weise wie oben beschrieben. Die Belichtung erfolgt im Suntest-Schnellbelichtungsgerät, die Bestrahlungsdauer betrug 1 Stunde.

Die eingesetzten Photostabilisatoren führten in allen Fällen zu eindeutigen Haltbarkeitsverbesserungen.

Gallussäurepropylester zeigte bei Chlorprothixen-HCl,Clopenthixol-di-HCl, Flupentixol -di-HCl und Tiotixen-di-HCl den geringeren Stabilisierungseffekt. Diese schwächere Wirkung kann aus dem spektralen Verhalten abgeleitet werden: Die Lichtabsorption der Verbindung beginnt mit $\lambda$ 350 nm erst relativ spät und damit kommt im schutzbedürftigen Spektralbereich keine optimale Überdeckung zustande.

Vanillin und 8-Hydroxychinolin erwiesen sich dagegen mit Restextinktionen zwischen 93 % und 98 % überwiegend als wirkungsvollere Stabilisatoren, wobei nur geringfügige Unterschiede zwischen der Schutzwirkung beider Hilfsstoffe zu beobachten waren. Aufgrund des früheren Absorptionsbeginns und des im Falle von Vanillin hohen Extinktionskoeffizienten zeigen diese Verbindungen sehr gute Abdeckeffekte. Zur Stabilisierung von Doxepin-HCl und erwies sich Gallussäurepropylester mit Restextinktionen von 99 % als gleichwertig.

– 14 –

<u>Patentansprüche:</u>

1. Verfahren zum Stabilisieren von photoinstabilen Arzneistoffen in Arzneiformen, dadurch gekennzeichnet, daß pharmakologische verträgliche, geeignete spektral absorbierende Substanzen zugegeben werden, welche ein ähnliches Absorptionsverhalten wie die zu schützenden Arzneistoffe aufweisen und vor dem jeweils speziellen, die Photoinstabilität auslösenden Spektralbereich des Lichtes schützen.

2. Verfahren gemäß Anspruch 1, dadurch gekennzeichnet, daß dem Arzneistoff oder der Arzneiform der Stabilisator in Form einer Lösung zugesetzt wird.

3. Verfahren gemäß Anspruch 1 oder 2, dadurch gekennezeichnet, daß man Dihydroergotamin, Nitrofurazon, Thiothixen, Furosemid, Piretanid, Nitroprussid-Natrium, Haloperidol, Fluphenazin, Reserpin, Rescinamin stabilisiert.

4. Verfahren gemäß Anspruch 1, dadurch gekennzeichnet, daß man Chlorpromazin-HCl, Protriptylin-HCl, Chlorprothixen-HCl, Clopenthixol-di-HCl, Flupentixol-di-HCl, Thiotixendi-HCl und Doxepin-HCl mit Vanillin, 8-Hydroxychinolin oder Gallussäurepropylester stabilisiert.

5. Verfahren gemäß Anspruch 2, dadurch gekennzeichnet, daß man als Stabilisator Vanillin, 8-Hydroxychinolin, Hydroxychinolinsalze, Gallussäurealkylester, Kurkumin, Benzylalkohol oder Lebensmittelfarbstoffe einzeln oder im Gemisch einsetzt   in Form von Lösungen.

6. Stabilisierte Arzneimittelform erhalten nach Verfahren gemäß Anspruch 1 oder 2.